# FASCICULE DE BREVET EUROPEEN

(11) **EP 0 876 813 B1**
(45) Date de publication et mention de la délivrance du brevet: **24.03.2004**
(21) Numéro de dépôt: 98400837.5
(22) Date de dépôt: 07.04.1998
(51) Int. Cl.: A61K 7/48, A61K 7/06, A61K 35/74

(54) **Composition cosmétique ou pharmaceutique comprenant un milieu de culture de micro-organisme**
Kosmetische oder pharmazeutische Zusammensetzung, die ein Kulturmedium eines Mikroorganismus enthält
Cosmetic or pharmaceutical composition containing a microorganism culture medium

(30) Priorité: 05.05.1997 FR 9705510
(43) Date de publication de la demande: 11.11.1998
(73) Titulaire: L'OREAL, 75008 Paris (FR)
(72) Inventeur: Pineau, Nathalie, 86000 Poitiers (FR); Martin, Richard, 37210 Rochecorbon (FR); Breton, Lionel, 78000 Versailles (FR); Aubert, Lucien, 06320 Cap D'Ail (FR)
(74) Mandataire: Tezier Herman, Béatrice

(56) Documents cités:
- EP-A- 0 761 204
- EP-A- 0 765 667
- WO-A-94/02158
- GB-A- 2 034 687

## Description

La présente invention concerne une composition cosmétique ou pharmaceutique comprenant à titre de principe actif une quantité efficace de milieu de culture d'au moins une bactérie filamenteuse non photosynthétique, ledit milieu étant clarifié et stabilisé. Elle concerne également l'utilisation dudit milieu de culture ainsi qu'un procédé de traitement cosmétique utilisant ladite composition.

Il est connu de l'art antérieur l'utilisation en cosmétique ou en pharmacie de micro-organismes, principalement sous forme d'extraits, pour les propriétés qui ont pu leur être reconnues.
Par extrait, on entend ici un produit correspondant à une biomasse de micro-organismes qui, après culture et une fois séparée de son milieu de culture, subit divers traitements pouvant aller de la simple congélation de ladite biomasse jusqu'à des purifications très élaborées de constituants des micro-organismes.

On peut par exemple citer les extraits de levure utilisés en cosmétique, les bactéries utilisées pour la préparation de produits laitiers, les extraits bactériens utilisés comme bactéricides, comme cicatrisants ou encore comme immunostimulants.
Plus précisément, on peut citer l'utilisation en cosmétique de fractions ribosomales par exemple dans la préparation de compositions destinées à retarder le vieillissement de la peau par stimulation de la croissance cellulaire et modulation de la maturation du tissu conjonctif (EP-A-631773).

En médecine, on connaît des compositions destinées à renforcer les défenses immunitaires en particulier de patients ayant subi de graves brûlures et sensibles de ce fait aux infections opportunistes causées par des bactéries, des virus ou des champignons (WO 9111174) ou encore en ce qui concerne les maladies de la sphère otorhinolaryngologique (FR 2253499, FR 2360314, FR 2388563, FR 2674755, ZA 8801071) ou encore les allergies (US 4946945).
Ces compositions utilisent en général des préparations de fractions ribosomales de micro-organismes.

Il est une classe de bactéries pour laquelle de nombreuses propriétés ont été mises en évidence et qui sont utilisées tant en cosmétique qu'en pharmacie. Il s'agit de la classe des bactéries filamenteuses non photosynthétiques telles que définies selon la classification du Bergey's Manual of Systematic Bacteriology (vol. 3, sections 22 et 23, 9°édition, 1989).
Ces bactéries, dont plusieurs ont déjà été décrites, ont généralement un habitat aquatique et peuvent être trouvées notamment dans des eaux marines ou dans des eaux thermales.

Ces bactéries, ou leurs extraits, sont décrits dans le brevet FR-2 283 223 pour leurs propriétés anti-inflammatoire, cicatrisante, leur pouvoir anti-acnéique, anti-séborrhéique, leur capacité à favoriser l'hydratation cutanée.

Un effet bactériostatique de la biomasse et du milieu de culture de Beggiatoa est connu par GB 2 034 687.

La demande EP-A-0681831 décrit l'utilisation de ces bactéries ou de leurs extraits dans des compositions cosmétiques destinées à lutter contre le vieillissement cutané. En effet, ces bactéries ou leurs extraits ont montré des propriétés dans le domaine du renouvellement épidermique.

La demande EP-A-0761204 revendique l'utilisation de ces bactéries ou de leurs extraits pour leur propriété apaisante des peaux sensibles, propriété d'ailleurs due à leur propriété d'antagoniste de substance P.

Dans la demande WO-9402158 et dans la demande EP-A-0765667 les propriétés immunostimulantes de ces bactéries ou de leurs extraits sont revendiquées en cosmétique et en pharmacie.

Dans toutes ces demandes, l'agent actif est constitué par la biomasse de bactéries, séparée de son milieu de culture et ayant éventuellement subi différents traitements.
L'obtention de cette biomasse nécessite des conditions de croissances particulières qui la rendent délicate et accroissent le temps nécessaire à la préparation ainsi que le prix de revient de cette matière première.

De manière surprenante et inattendue, la demanderesse a découvert que le milieu de culture des bactéries filamenteuses non photosynthétiques, clarifié et stabilisé, jusqu'alors considéré comme inutilisable en fin de culture et éliminé, présente des propriétés similaires de celles de la biomasse. De plus, de manière encore plus inattendue le niveau d'expression de ces propriétés par le milieu est tout à fait comparable à celui de la biomasse.
On comprend aisément l'intérêt que représente une telle découverte, en particulier quand on sait qu'une culture en fermenteur de 300 litres n'aboutit qu'à la production de biomasse comprise entre 270 grammes et 360 grammes poids sec maximum.

Ainsi, l'invention à pour objet une composition cosmétique ou pharmaceutique comprenant, dans un support cosmétiquement et/ou pharmaceutiquement acceptable, une quantité efficace de milieu de culture d'au moins une bactérie filamenteuse non photosynthétique, ledit milieu étant clarifié et stabilisé.

Par support cosmétiquement et/ou pharmaceutiquement acceptable, on entend tout support physiologiquement acceptable.

Par milieu de culture clarifié, on entend un milieu de culture ayant servi à l'amplification d'un micro-organisme, en particulier d'une bactérie, qui après amplification dudit micro-organisme a subi une manipulation consistant en la séparation physique dudit milieu de culture et dudit micro-organisme.

Par milieu de culture stabilisé, on entend un milieu de culture ayant subi une manipulation destinée à la préserver dans l'état dans lequel il se trouvait à un instant donné choisi, tout en ayant conservé ses propriétés intrinsèques. En particulier, s'agissant d'un milieu de culture de micro-organismes, cette manipulation est destinée, par exemple, à rendre ledit milieu stérile, c'est à dire incapable de permettre la croissance de micro-organismes tout en préservant, par exemple, les éventuelles propriétés biologiques dont il est doté.

Parmi les bactéries filamenteuses non photosynthétiques utilisées selon l'invention, les bactéries appartenant à l'ordre des Beggiatoales sont particulièrement préférées. Encore plus particulièrement, on préfère les bactéries appartenant aux genres Beggiatoa, Vitreoscilla, Flexithrix ou Leucothrix.

Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

Préférentiellement, on utilise selon l'invention une souche de *Vitreoscilla filiformis*.

Pour préparer le milieu de culture utilisable selon l'invention, on peut cultiver lesdites bactéries selon les méthodes connues de l'homme du métier. A cet égard on trouvera dans le Bergey's Manual of Systematic Bacteriology (*9°édition, 1989*) toutes les informations nécessaires à la culture de ces bactéries.

On sait que la culture des bactéries filamenteuses non photosynthétiques est relativement difficile, de même que l'obtention de cultures pures. La plupart des auteurs préconisent l'utilisation de milieux mal définis, y compris des macérations diverses utilisant l'eau de ville. La source de carbone recommandée est un acétate.

Préférentiellement, le milieu de culture utilisable dans l'invention est le milieu de culture ayant permis la préparation d'une biomasse de bactéries filamenteuses non photosynthétiques selon le procédé décrit par la demanderesse dans la demande de brevet WO-9402158.

En effet, la demanderesse a montré qu'il est possible d'adapter ces bactéries, par contre-sélection, à l'utilisation d'un ose, au lieu d'acétate, comme source de carbone.
Elle a en outre montré qu'il est possible de cultiver ces bactéries sur un milieu de culture parfaitement défini. On peut en particulier effectuer une culture dans le milieu suivant:

| COMPOSITION | CONCENTRATION |
|---|---|
| Extrait autolytique de levures | 0,5 à 5 g/l |
| Peptone | 0,5 à 5 g/l |
| Glucose anhydre | 0,5 à 7 g/l |
| Micro-éléments de Heller | 0,5 à 5 ml/l |
| CaCl₂, 10 H₂O | 0,01 à 0,20 g/l |

On complète à 1000 ml par de l'eau distillée. Parmi les peptones utilisables, on peut citer par exemple la peptone papaïnique de soja.
Ce milieu particulier se distingue des milieux généralement utilisés par l'absence de catalase et de sulfure, alors que ces ingrédients étaient généralement considérés comme indispensables jusqu'à présent.

Les micro-éléments de Heller, dont la composition est donnée ci-après, ont été décrits par Heller, Ann Sci. Nat. Biol. Veg. 14: 1-223 (1953).

Il s'agit de mélanges de divers éléments minéraux qui ont été recommandés par Heller, non pas pour la culture des bactéries, mais pour la nutrition des tissus végétaux cultivés *in vitro*. Il convient de noter ici que l'on n'a pas cherché à déterminer si les micro-éléments de Heller sont tous indispensables ou utiles dans la culture des bactéries filamenteuses non photosynthétiques. Il a cependant été trouvé que les micro-éléments de Heller utilisés ensemble en combinaison avec les autres constituants mentionnés dans le tableau précédent permettent effectivement la culture des bactéries considérées.

La composition des micro-éléments de Heller, pour 1 litre d'eau distillée, est la suivante :

| | |
|---|---|
| ZnSO₄, 7 H₂O | 1g |
| MnSO₄, H₂O | 0,076 g |
| CuSO₄, 5H₂O | 0,003 g |
| Kl | 0,010 g |
| H₃BO₃ | 1 g |
| AlCl₃, 6H₂O | 0,050 g |
| NiCl₂, 6H₂O | 0,030 g |

La culture peut être effectuée à la température appropriée convenant pour l'espèce bactérienne cultivée. Généralement, cette température est comprise entre 18 et 40°C suivant les souches. Le pH du milieu de culture est de préférence compris entre 5,5 et 8.

Lorsque la culture bactérienne à atteint un stade terminal, c'est à dire en général lorsque la biomasse est en fin de croissance exponentielle, on sépare ladite biomasse du milieu de culture. Cette séparation peut être obtenue par toute technique classique comme par exemple la centrifugation, la filtration, la coagulation avec un alcool (éthanol, isopropanol, isobutanol) ou le séchage sur cylindre à précouche (amidon, diatomées...) raclée.

Préférentiellement, on utilise la méthode de la centrifugation.

La stabilisation du milieu de culture peut alors être réalisée par tout procédé connu. A titre d'exemple, on cite la filtration stérilisante, l'autoclavage, l'ultra haute température (technique UHT), la stérilisation haute pression, les rayonnements y ou la congélation.

Préférentiellement, on utilise la méthode de stabilisation par autoclavage. Avantageusement, l'autoclavage est réalisé à une' température comprise entre 115°C et 121° C et de préférence pendant un temps compris entre 15 et 40 minutes.

Un exemple de préparation du milieu de culture utilisable selon l'invention est donné par ailleurs dans les exemples.

La quantité de milieu de culture d'au moins une bactérie filamenteuse non photosynthétique, clarifié et stabilisé utilisable dans la composition de l'invention est bien entendu fonction de l'effet recherché. Elle peut donc varier dans une large mesure.

Pour donner un ordre de grandeur la composition peut contenir une quantité de milieu de culture d'au moins une bactérie filamenteuse non photosynthétique représentant de 0,0001% à 30% du poids total de la composition et préférentiellement en une quantité représentant de 0,01 % à 15% du poids total de la composition.

La composition peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention, la composition peut comprendre d'autres agents actifs destinés notamment à la prévention et/ou au traitement des affections cutanées. Parmi ces agents actifs, on peut citer à titre d'exemple :
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides α- et β-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'a-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, la composition selon l'invention peut comprendre également au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération cellulaire et/ou la pigmentation cutanée.

On sait que le vieillissement cutané, qu'il soit dû à l'âge ou à d'autres facteurs tels que les facteurs d'environnement, se traduit notamment par une détérioration des propriétés mécaniques de la peau, et en particulier une perte d'élasticité et de tonicité avec apparition de rides. On rattache ce phénomène notamment à une altération des tissus élastiques et en particulier à une diminution du nombre et du diamètre des fibres élastiques. Le vieillissement cutané s'accompagne également d'un amincissement de l'ensemble des composants de la peau avec pour conséquence une augmentation de la fragilité cutanée. La raréfaction des fibroblastes ainsi que l'altération de leur activité sont considérées comme jouant un rôle très important dans le processus de vieillissement cutané. On sait également que des échanges gazeux se produisent à la surface de la peau avec élimination de dioxyde de carbone et absorption d'oxygène. Ce phénomène, appelé respiration cutanée, diminue avec l'âge et cette diminution est considérée comme une résultante de la diminution de l'activité épidermique.

La demanderesse a trouvé que le milieu de culture d'au moins une bactérie filamenteuse non photosynthétique, clarifié et stabilisé, est capable de diminuer et/ou de retarder le vieillissement cutané, lorsqu'il est appliqué sur la peau.

Particulièrement, la demanderesse a trouvé que le milieu de culture est capable de modifier les échanges gazeux au travers de la peau, de stimuler la prolifération des fibroblastes et d'améliorer ainsi les propriétés mécaniques de l'épiderme.

Ainsi, l'invention concerne également un procédé de traitement cosmétique destiné à diminuer et/ou de retarder le vieillissement cutané, tel que l'on applique sur la peau, sur le cuir chevelu et/ou sur les muqueuses une composition cosmétique telle que décrite précédemment.

L'invention a aussi pour objet une composition pharmaceutique destinée à diminuer et/ou à retarder le vieillissement cutané comprenant une quantité efficace de milieu de culture d'au moins une bactérie filamenteuse non photosynthétique tel que décrit précédement.

Le système immunitaire comprend un ensemble de cellules spécialisées soumises à de multiples mécanismes de contrôle assurant leur renouvellement, leur activation et leur différenciation, indispensables à un niveau normal d'immunocompétence. Le rôle du système immunitaire est de discriminer le soi du non soi pour éliminer les agents pathogènes et les tumeurs spontanées. Toute déplétion cellulaire, toute dysrégulation immunitaire ou tout déficit fonctionnel est susceptible de favoriser la survenue de manifestations pathologiques caractérisées par la perturbation des mécanismes de reconnaissance du soi vis-à-vis du non soi, et une plus grande sensibilité vis-à-vis des agressions microbiennes et des processus néoplasiques.

La peau constitue l'organe le plus important de l'organisme et est reconnue comme l'un des principaux éléments actifs du système de défense immunitaire. Trois types de cellules épidermiques participent à ce système : les kératinocytes, les mélanocytes et les cellules de Langerhans. Ces cellules, que l'on ne retrouve qu'au niveau de la peau, jouent un rôle primordial dans la réponse immunitaire et en particulier dans la présentation antigénique.

La peau saine est capable de se défendre des agressions extérieures grâce aux moyens mis à sa disposition. Néanmoins, elle est soumise à l'agression permanente de l'environnement, de produits chimiques et de radiations. En particulier, les cellules de Langerhans sont la cible privilégiée des rayonnements ultraviolets.

Ces agressions se traduisent par un effet suppresseur des défenses immunitaires entraînant une baisse des résistances aux agents pathogènes et une augmentation de l'incidence de certains cancers.

Pour aider la peau à remplir sa fonction immunitaire, des produits de stimulation du système immunitaire cutanée sont d'un grand intérêt.

On sait d'autre part que le système immunitaire et plus particulièrement celui de la peau s'affaiblit au cours du vieillissement chronobiologique.
Cet affaiblissement survient également au cours du vieillissement photo-induit.
Un effet immunostimulateur peut alors rétablir les fonctions immunitaires et plus particulièrement celles de l'épiderme en renforçant les défenses naturelles de la peau.

La demanderesse a trouvé que l'application d'une quantité efficace de milieu de culture d'au moins une bactérie filamenteuse non photosynthétique, clarifié et stabilisé, permet de stimuler le système immunitaire, plus particulièrement le système immunitaire de la peau.

Particulièrement, le milieu ou la composition est destiné à stimuler les défenses immunitaires au cours du vieillissement chronobiologique, ainsi qu'au cours du vieillissement photo-induit.

L'invention a donc également pour objet un procédé de traitement cosmétique destiné à stimuler les défenses immunitaires, tel que l'on applique sur la peau, sur les cheveux et/ou sur les muqueuses une composition cosmétique telle que décrite précédemment. Particulièrement ce procédé de traitement cosmétique est destiné à stimuler les défenses immunitaires au cours du vieillissement chronobiologique ainsi qu'au cours du vieillissement photo-induit.

L'invention a aussi pour objet une composition pharmaceutique destinée à stimuler le système immunitaire, comprenant une quantité efficace de milieu de culture d'au moins une bactérie filamenteuse non photosynthétique tel que décrit précédement.

Il existe chez les mammifères des polypeptides appartenant à la famille des tachykinines qui induisent sur les fibres musculaires lisses des contractions rapides. Parmi les composés de cette famille on peut citer la neurokinine β, neurokinine α et la substance P.

La substance P est un élément chimique polypeptidique (undécapeptide), élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment un système nerveux central et périphérique.

De par la distribution ubiquitaire de la substance P, de très nombreux désordres sont associés à un excès de synthèse et / ou de libération de substance P.

La substance P intervient notamment dans la transmission de la douleur et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie), dans des maladies respiratoires (telles que par exemple les broncho-pneumonies) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn), dans des désordres cutanés (tels que par exemple le psoriasis, les maladies prurigineuses, l'herpès, les photodermatoses, les dermatites atopiques, les dermatites de contact, les lichens, les prurigos, les prurits, les piqûres d'insectes), dans des fibroses et autres troubles de la maturation des collagènes (tels que par exemple la sclérodermie), dans des troubles cardio-vasculaires, dans des troubles vasospastiques (tels que par exemple les migraines, la maladie de Reynaud), dans des désordres immunologiques, dans des troubles du tractus urinaire (tels que par exemple l'incontinence, la cystite), dans des maladies rhumatismales dans certaines maladies dermatologiques (telles que l'eczéma) et dans les affections ophtalmologiques (telles que par exemple les conjonctivites, les uvéites, les prurits oculaires, les douleurs oculaires et les irritations).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P.
Particulièrement, pour qu'une substance soit reconnue comme un antagoniste de substance P elle doit induire une réponse pharmacologique cohérente (incluant ou non sa fixation au récepteur de la substance P) notamment dans l'un des tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

La demanderesse a trouvé que le milieu de culture d'au moins une bactérie filamenteuse non photosynthétique, clarifié et stabilisé, répond aux caractéristiques définies comme caractérisant un antagoniste de substance P et peut donc être utilisé comme antagoniste de substance P.

Ainsi, l'invention concerne l'utilisation d'une quantité efficace de milieu de culture d'au moins une bactérie filamenteuse non photosynthétique, ledit milieu étant clarifié et stabilisé, comme antagoniste de substance P pour la préparation d'une composition cosmétique ou d'une composition pharmaceutique.

L'invention concerne également un procédé de traitement cosmétique destiné à traiter les désordres associées à un excès de synthèse et/ou de libération de substance P, tel que l'on applique sur la peau, sur le cuir chevelu et/ou sur les muqueuses une composition cosmétique telle que décrite précédemment.

L'invention a aussi pour objet une composition pharmaceutique destinée à traiter les désordres associées à un excès de synthèse et/ou de libération de substance P, comprenant une quantité efficace de milieu de culture d'au moins une bactérie filamenteuse non photosynthétique tel que décrit précédement.

En outre, l'invention a aussi pour objet une composition pharmaceutique destinée à traiter les désordres du système nerveux central, les troubles respiratoires, les syndromes allergiques, l'inflammation, la douleur, les désordres gastro-intestinaux, les désordres cutanés, les fibroses, les troubles de la maturation du collagène les troubles cardio-vasculaires, les troubles vasospastiques, les désordres immunologiques et/ou les troubles du tractus urinaires, comprenant une quantité efficace de milieu de culture d'au moins une bactérie filamenteuse non photosynthétique tel que décrit précédement.

Dans le domaine des désordres cutanés, il est connu que certaines peaux sont plus sensibles que d'autres. On sait qu'il existe au niveau cutané de nombreux phénomènes d'intolérance dont les symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

Ces phénomènes peuvent être la conséquence d'événements multiples dont les plus banaux seront qualifiés d'irritation ou d'inflammation, mais dont certains seront dus à des causes physiologiques, comme les peaux sensibles, voire même pathologiques comme par exemple, l'allergie.

Toutefois les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.

Des tests ont été mis au point pour cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles.

La demanderesse a trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont associés à une peau hyperséborrhéique ou acnéique avec ou sans dartres, et à un érythème.

Ces phénomènes peuvent être généralisés à l'ensemble du corps, mais la plupart du temps ils peuvent avoir des localisations bien définies telles que par exemple le cuir chevelu, le visage, les plis cutanés, etc....

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.

L'ensemble de ces phénomènes d'intolérance est toujours lié à un processus inflammatoire classique, et plus particulièrement à une réaction inflammatoire de type neurogène puisqu'elle fait intervenir les fibres nerveuses cutanées.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. Par contre, la résultante finale d'une réaction allergique se traduit également par une réaction inflammatoire aiguë associé généralement à un oedème.

La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

Quelque soit le phénomène envisagé, il existe un point commun à tous ces mécanismes qui se traduit par une réaction inflammatoire dont la facette terminale se mesure par la libération par les cellules mastocytaires de la peau d'au moins un médiateur de l'inflammation tels que l'histamine, la sérotonine, l'héparine, les leukotriènes, les prostaglandines, les cytokines, le monoxyde d'azote ou des espèces oxygénées réactives.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Personne jusqu'à ce jour n'avait établi un lien entre la substance P et la peau sensible. Les signes cliniques de la peau sensible sont essentiellement subjectifs : picotements, fourmillements, prurits,. tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Ainsi la demanderesse a découvert que l'une des caractéristiques essentielles des peaux sensibles est liée à la libération de substance P et donc que l'utilisation d'antagonistes de substance P peut permettre d'obtenir un effet préventif et/ou curatif des peaux sensibles.

Pour traiter les peaux sensibles, la demanderesse a donc envisagé d'utiliser des antagonistes de substance P. Elle a en effet constaté de manière surprenante que l'incorporation d'un antagoniste de substance P dans une composition destinée à un usage topique permet d'éviter l'irritation et/ou les sensations dysesthésiques et/ou les prurits de la peau.

L'invention concerne donc plus particulièrement un procédé de traitement cosmétique destiné à traiter les peaux sensibles, tel que l'on applique sur la peau, sur le cuir chevelu et/ou sur les muqueuses une composition cosmétique telle que décrite précédemment.

L'invention a aussi pour objet une composition pharmaceutique destinée à traiter les peaux sensibles comprenant une quantité efficace de milieu de culture d'au moins une bactérie filamenteuse non photosynthétique tel que décrit précédemment.

La présente invention a encore pour objet l'utilisation d'une quantité efficace de milieu de culture d'au moins une bactérie filamenteuse non photosynthétique, ledit milieu étant clarifié et stabilisé, dans une composition cosmétique ou pour la préparation d'une composition pharmaceutique, ledit milieu ou ladite composition étant destiné à prévenir et/ou à lutter contre les irritations cutanées et/ou les dartres et/ou les érythèmes et/ou les sensations d'échauffement et/ou de dysesthésie et/ou les prurits de la peau et/ou les muqueuses.

L'invention a également pour objet un procédé de traitement cosmétique destiné à prévenir et/ou à lutter contre les irritations cutanées et/ou les dartres et/ou les érythèmes et/ou les sensations d'échauffement et/ou de dysesthésie et/ou les prurits de la peau et/ou les muqueuses, tel que l'on applique sur la peau, sur le cuir chevelu et/ou sur les muqueuses une composition cosmétique telle que décrite précédemment.

De façon avantageuse selon l'invention, une quantité efficace de milieu de culture d'au moins une bactérie filamenteuse non photosynthétique, ledit milieu étant clarifié et stabilisé, peut être associé à des produits à effet irritant utilisés couramment dans le domaine cosmétique ou pharmaceutique, produits qui sont parfois des actifs cosmétiques ou pharmaceutiques.

La présence d'un antagoniste de substance P sous la forme d'une quantité efficace de milieu de culture d'au moins une bactérie filamenteuse non photosynthétique, ledit milieu étant clarifié et stabilisé, dans une composition cosmétique ou pharmaceutique comprenant un produit ayant un effet irritant permet d'atténuer fortement, voire de supprimer cet effet irritant.
Cela permet en outre d'augmenter la quantité d'actif à effet irritant par rapport à la quantité d'actif normalement utilisée, en vue d'une efficacité améliorée.

Ainsi, l'invention concerne également l'utilisation d'une quantité efficace de milieu de culture d'au moins une bactérie filamenteuse non photosynthétique, ledit milieu étant clarifié et stabilisé, dans une composition cosmétique ou pharmaceutique comprenant en outre au moins un produit à effet irritant.

L'invention concerne également une composition cosmétique ou pharmaceutique comprenant dans un milieu cosmétiquement ou pharmaceutiquement acceptable une quantité efficace de milieu de culture d'au moins une bactérie filamenteuse non photosynthétique, ledit milieu étant clarifié et stabilisé, et en outre au moins un produit à effet irritant.
Comme produits à effet irritant on peut citer par exemple les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxy-acides (l'acide salicylique et ses dérivés), les α-céto-acides, les β-céto-acides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, les kératolytiques, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

L'emploi d'antagoniste de substance P permet notamment de multiplier de 2 à 10 fois la quantité d'actif à effet irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, en diminuant notablement leur caractère irritant.

La demanderesse a trouvé que le milieu de culture d'au moins une bactérie filamenteuse non photosynthétique, clarifié et stabilisé, présente des propriétés anti-inflammatoire, cicatrisante, anti-acnéique, anti-séborréhique, et qu'il favorise l'hydratation cutanée.

Ainsi, les compositions selon l'invention peuvent aussi servir à apaiser l'inflammation, à favoriser la cicatrisation, à diminuer l'acné et/ou la séborrhée et/ou à favoriser l'hydratation cutanée, tel que l'on applique sur la peau, sur les cheveux et/ou sur les muqueuses une composition cosmétique telle que décrite précédemment.

L'invention a aussi pour objet une composition pharmaceutique destinée à apaiser l'inflammation, à favoriser la cicatrisation, à diminuer l'acné et/ou la séborrhée et/ou à favoriser l'hydratation cutanée comprenant une quantité efficace de milieu de culture d'au moins une bactérie filamenteuse non photosynthétique tel que décrit précédemment.

Les procédés de traitement cosmétique de l'invention peuvent être mis en oeuvre notamment en appliquant les compositions hygiéniques ou cosmétiques telles que définies ci-dessus, selon la technique d'utilisation habituelle de ces compositions : par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention. Dans les compositions, les proportions indiquées sont des pourcentages en poids sauf mention contraire.

### Exemple 1 : Préparation de milieu de culture d'au moins une bactérie filamenteuse non photosynthétique clarifié et stabilisé :

Une souche de *Vitreoscilla filiformis* (ATCC 15551) est mise en culture selon le procédé décrit dans la demande de brevet WO-9402158. La culture s'effectue à 26°C durant au moins 48 heures jusqu'à l'obtention d'une concentration cellulaire convenable correspondant à une densité optique à 600 nm supérieure ou égale à 1,5. On repique la souche à 2 % VN dans du milieu neuf toutes les 48 heures jusqu'à l'obtention d'une culture stable. Un Erlenmeyer de 1 litre contenant 200 ml de milieu neuf est alors ensemencé avec 4 ml de la culture précédente.

La culture en Erlenmeyer s'effectue à 26° C sur une table de culture agitée à 100 tours/minutes. Le pied de cuve ainsi obtenu sert d'inoculum à un fermenteur de 10 I. La croissance s'effectue à 26° C, pH 7, 100 tours/minute et pO₂≥ 15 %.

Après 48 heures de croissance, la biomasse est transférée dans un fermenteur de 600 litres utiles, pour être cultivée dans les mêmes conditions.

On a utilisé le milieu de culture suivant :

| COMPOSITION | CONCENTRATION |
|---|---|
| Extrait autolytique de levure Biokar (réf. 112002) | 2,0 g/l |
| Peptone papaïnique de soja (origine PPS-USP Biokar réf. 1 1601) | 2,0 g/l |
| Micro-éléments de Heller | 2,0 ml/l |
| Glucose anhydre | 2,0 g/l |
| CaCl₂, 10 H₂O | 0,066 g/l |
| Eau distillée | 100,0 ml |

Le pH est ajusté à 7,15 par addition de soude ou potasse 1N avant stérilisation à 121 °C pendant 20 min.

La composition des micro-éléments de Heller, pour 1I d'eau distillée, est la suivante :

| | |
|---|---|
| ZnSO₄, 7 H₂O | 1g |
| MnSO₄, H₂O | 0,076 g |
| CuSO₄, 5H₂O | 0,003 g |
| Kl | 0,010 g |
| H₃BO₃ | 1 g |
| AlCl₃,6H₂O | 0,050 g |
| NiCl₂,6H₂O | 0,030 g |

On ajoute à ce milieu de culture 0,2 g/l d'un antimousse de type polyméthylsiloxane (Silbione 97350 RP). La température est régulée entre 26 et 30°C, l'optimum se situant à 29°C.

Un cycle complet de croissance s'effectue en 48h environ.

L'aération est régulée par un débitmètre massique pour avoir au minimum 20 % d'oxygène dissous.

Il n'y a pratiquement plus de glucose résiduel en fin de croissance.
La séparation de la biomasse est effectuée par centrifugation.
On opère dans une centrifugeuse de type industriel refroidie à 4°C permettant d'obtenir un pouvoir séparatif équivalent à 8000 g, imprimé pendant 2 minutes.

Le milieu de culture ainsi récolté peut ensuite être stocké par congélation pour une utilisation différée.

Avant utilisation le milieu est stérilisé à une température comprise entre 114°C et 121°C pendant 15 à 40 minutes.

### Exemple 2 : On étudie dans cet exemple la capacité du milieu de l'exemple 1 à stimuler les défenses immunitaires.

### A) Etude de la stimulation des splénocytes de souris.

Cette étude est réalisée selon le protocole décrit par L.E. Averill et N.S. Wolf (1985) Journal of Immunology 134 : 3859-3863.

### Préparation des splénocytes de souris

Des rates de souris Balb/C sont dilacérées dans du milieu RPMI 1640 (SIGMA). La suspension cellulaire est filtrée sur un filtre de type "cell stainer" de la société Nunc afin d'éliminer les agrégats cellulaires. La suspension est ensuite centrifugée à 1000 RPM pendant 10 minutes à 4°C. Le culot cellulaire est suspendu dans du milieu RPMI complet contenant de la L-glutamine à 2 mM, de la pénicilline à 100 unités/ml, de la streptomycine à 100 µg/ml, du 2-mercaptoethanol à 5.10⁻⁵ M et du sérum de veau foetal à 10%. La suspension cellulaire est ajustée à 2.5 x 10⁶ cellules par ml.

### Test de prolifération cellulaire :

Les cellules telles que précédemment préparées sont réparties dans des plaques de culture 96 puits (FALCON) à raison de 2.5 x 10⁵ cellules par puits, dans un volume final de 100 µl. 20 µl de milieu de culture d'au moins une bactérie filamenteuse non photosynthétique, clarifié et stabilisé de l'exemple 1 sont ajoutés aux concentrations finales à étudier. Le volume de chaque puits est ajusté à 200 µl par addition de 80 µl de milieu RPMI complet. Les cellules sont cultivées pendant 48 et 72 heures. 18 heures avant la fin de la culture, 1 µCi de thymidine tritiée d'activité spécifique égale à 82 Ci/mmole (Amersham) est ajoutée dans chaques puits. A la fin du temps de culture, les cellules sont récupérées sur filtre. Après séchage du filtre celui-ci est introduit dans un compteur β à scintillation liquide afin de déterminer la radioactivité incorporée au niveau de l'ADN.

Pour comparaison des effets, le même test a été effectué sur des parois de *Vitreoscilla filiformis.* Ces parois ont été préparées à partir de la biomasse obtenue après culture et séparation du milieu, par traitement à 121°C pendant 15 minutes. Ces parois sont connues pour présenter un effet immunostimulant (WO-9402158).

La mesure de la radioactivité incorporée à l'ADN permet de déterminer le taux de croissance des splénocytes.

Les résultats sont présentés dans le tableau suivant :

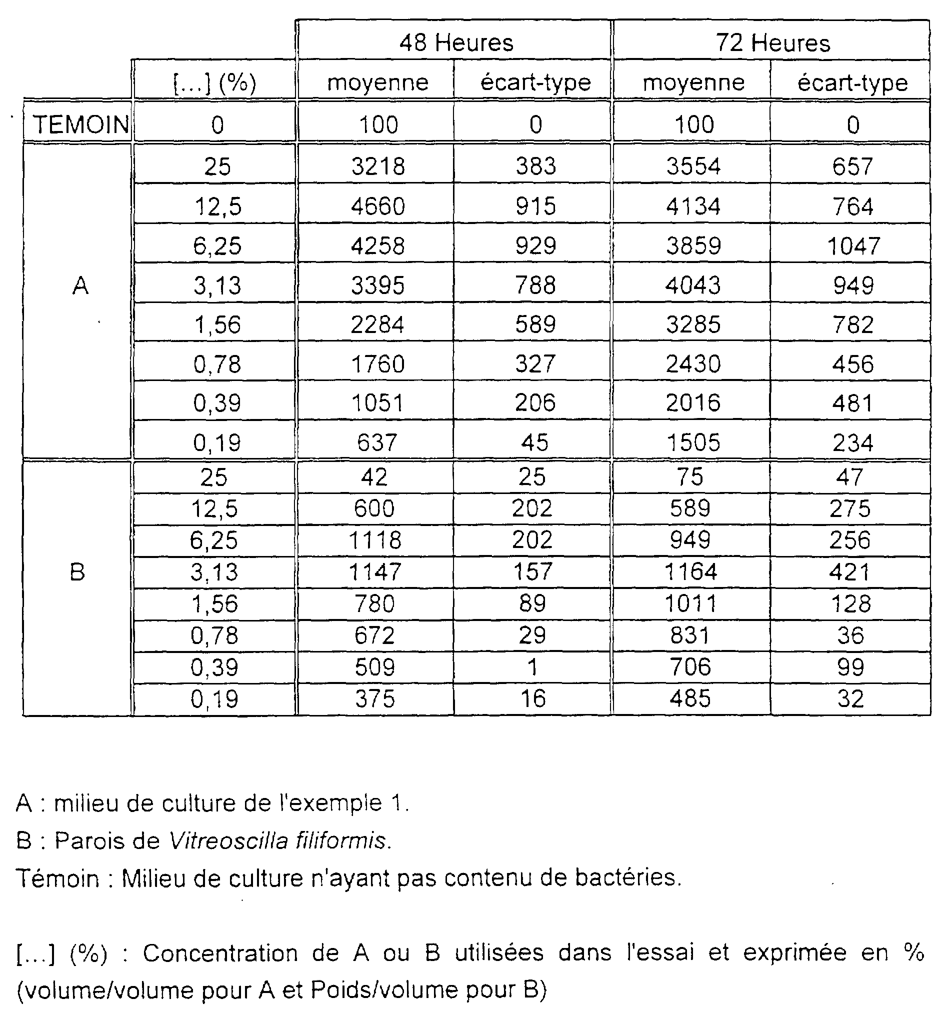

Les résultats sont exprimés en indice de stimulation calculé selon la formule : valeur de l'essai / valeur du témoin x 100.
Les valeurs supérieures à 20 sont considérées comme positives.

Le milieu de l'exemple 1 induit la croissance des splénocytes de souris. Le maximum de prolifération est obtenu à 48 heures de culture pour la concentration de 12,5 %. Les résultats sont supérieurs à ceux obtenus avec les parois de *Vitreoscilla filiformis.*

### B) Etude de la production d'immunoglobulines

Des cellules telles que préparées dans l'exemple 2 sont réparties dans des plaques de culture 6 puits (FALCON) à raison de 10 x 10⁶ cellules par puits (volume de 2 ml par puits).
300 µl des différents produits à tester sont ajoutés aux concentrations à étudier, ce qui suppose qu'ils sont 10 fois concentrés. Le volume de chaque puits est ajusté à 3 ml par addition de 0.7 ml de milieu RPMI 1640 complet. Les cellules sont cultivées 3 jours puis les surnageants de culture sont prélevés. Le dosage des immunoglobulines est réalisé à l'aide du «Mab-based mouse lg isotyping kit» (PHARMINGEN) selon le protocole du fournisseur.

Les immunoglobulines sont des protéines présentes dans le sang qui se combinent aux antigènes afin que ces derniers soient reconnus et identifiés comme corps étranger par les macrophages.

Les immunoglobulines sont subdivisées en différents types et sous-types en fonction de la structure de la partie constante de leur chaîne lourde. Chez la souris, on distingue 5 classes (Ig M, G, A, D et E). L'immunoglobuline G est l'immunoglobuline la plus abondante dans les fluides corporels notamment extra-vasculaires où elle combat microorganismes et toxines. On peut classer les immunoglobulines G en 4 sous-classes (1, 2, 3 et 4).

L'immunoglobuline A est l'immunoglobuline majeure dans les sécrétions séro-muqueuses où elle défend les surfaces externes du corps.

L'immunoglobuline M est un agent agglutinant très efficace produit très tôt dans la réponse immunitaire. Elle constitue la première ligne de défense contre les bactériémies.

### Seules les Ig G1, G2a, G2b, G3, M et A ont été dosées dans ce test.

Pour comparaison des effets, le même test a été effectué sur des parois de *Vitreoscilla filiformis.* Ces parois ont été préparées à partir de la biomasse obtenue après culture et séparation du milieu, puis traitées thermiquement à 121°C pendant 15 minutes.
Un contrôle positif est réalisé avec des lipopolysaccharides (LPS) d'Escherichia coli connus pour être de bons immunostimulateurs.

Les résultats sont présentés dans le tableau suivant:

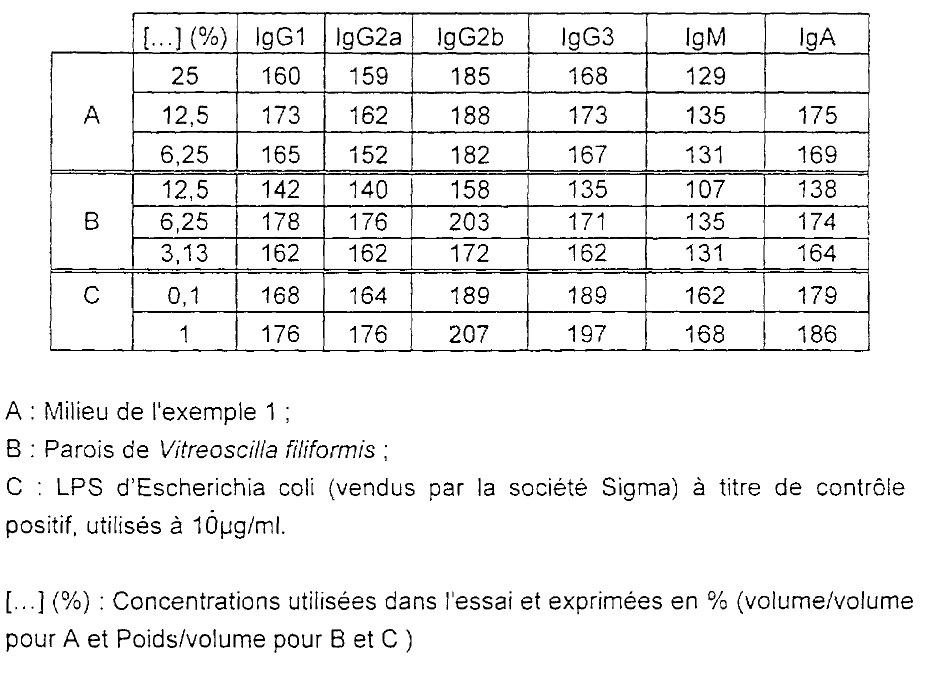

Les résultats sont exprimés en indice de production calculé selon la formule : valeur de l'essai / valeur du témoin x 100.
Les valeurs supérieures à 150 sont considérées comme positives.

Le milieu clarifié et stabilisé induit la production d'immunoglobulines par les cellules B.

Le milieu clarifié et stabilisé augmente la croissance des splénocytes et la production d'immunoglobulines. Aucune toxicité n'est observée avec ce milieu jusqu'à la dose de 25%.
Ces résultats démontrent bien le rôle immunomodulateur du milieu selon l'invention.

### Exemple 3 Exemples de formulations illustrant l'invention. Ces compositions ont été obtenues par simple mélange des différents composants.

### Composition 1 : Composition dermique sous forme de crème

| | |
|---|---|
| Milieu de l'exemple 1 | 2.0 g |
| Huile de Vaseline | 15.0 g |
| Huile de Tournesol | 5.0 g |
| Acide Stéarique | 2.0 g |
| Alcool Cétylique | 3.0 g |
| Stéarate de Polyéthylène Glycol à 100 OE | 5.0 g |
| Propylène Glycol | 3.0 g |
| Conservateurs | 0.3 g |
| Eau Purifiée | QSP 100.0 g |

Cette crème est appliquée à raison de 3 applications par jour sur des brûlures ou des plaies en vue d'accélérer la cicatrisation.

### Composition 2 : Lait après-soleil

| | |
|---|---|
| Milieu de l'exemple 1 | 1.0 g |
| Monostéarate de Glycérol auto-émulsionnable | 3.0 g |
| Huile de Vaseline | 4.0 g |
| Huile de Germes de Blé | 2.0 g |
| Huile de Silicone Volatile | 5.0 g |
| Beurre de Karité | 3.0 g |
| Carbomer 940* | 0.2 g |
| Triéthanolamine | 0.2 g |
| Gomme de xanthane | 0.1 g |
| Glycérine | 3.0 g |
| Composition parfumante | 0.1 g |
| Conservateurs | 0.3 g |
| Eau Purifiée | QSP 100.0 g |

| | |
|---|---|
| *Carbomer 940 = nom de commerce désignant un acide polyacrylique réticulé. | |

Ce lait est appliqué sur l'ensemble du corps après exposition solaire.

### Composition 3 : Composition anti-solaire

| | |
|---|---|
| Milieu de l'exemple 1 | 5.0 g |
| Acide Stéarique | 3.0 g |
| Alcool Cétylique | 1.6 g |
| Monostéarate de glycérol auto-émulsionnable | 3.0 g |
| Huile de Tournesol | 8.0 g |
| Polyacrylamide | 3.0 g |
| Octyl Méthoxyccinamate | 4.0 g |
| Sel de Triéthanolamine de l'acide téréphalylidène dicamphor | |
| sulfonique (Mexoryl SX) | 2.6 g |
| Glycérol | 5.0 g |
| Tocophérol | 2.0 g |
| Conservateurs | 0.3 g |
| Pentasodium Etylènediaminetétraméthylène Phosphonate | 0.1 g |
| Eau Purifiée | QSP 100.0 g |

Cette émulsion est utilisée pour protéger la peau des rayons ultraviolets et pour maintenir et stimuler le système immunitaire face au soleil.

## Revendications

1. Composition cosmétique ou pharmaceutique comprenant, dans un support cosmétiquement et/ou pharmaceutiquement acceptable, une quantité efficace de milieu de culture d'au moins une bactérie filamenteuse non photosynthétique, ledit milieu ayant servi à l'amplification dudit micro-organisme et étant clarifié et stabilisé.

2. Composition selon la revendication précédente, **caractérisée en ce que** ladite bactérie appartient à l'ordre des Beggiatoales.

3. Composition selon l'une quelconque des revendications précédentes, **caractérisée en ce que** ladite bactérie appartient au genre *Beggiatoa, Vitreoscilla*, *Flexithrix* ou *Leucothrix*.

4. Composition selon l'une quelconque des revendications précédentes, **caractérisée en ce que** ladite bactérie est choisie parmi des souches de *Vitreoscilla filiformis*.

5. Composition selon l'une quelconque des revendications précédentes, **caractérisée en ce que** le milieu représente de 0,0001% à 30% du poids total de la composition.

6. Composition selon la revendication précédente, **caractérisée en ce que** le milieu représente de 0,01% à 15% du poids total de la composition.

7. Composition selon l'une quelconque des revendications précédentes, **caractérisée en ce que** le milieu est stabilisé par filtration stérilisante, autoclavage, ultra haute température, stérilisation haute pression, rayonnement γ ou congélation.

8. Composition selon la revendication précédente, **caractérisée en ce que** le milieu est stabilisé par autoclavage.

9. Composition selon l'une quelconque des revendications précédentes, **caractérisée en ce que** le milieu comprend un acétate comme source de carbone.

10. Composition selon l'une quelconque des revendications 1 à 8, **caractérisée en ce que** le milieu comprend un ose comme source de carbone.

11. Composition selon l'une quelconque des revendications 1 à 8 et 10, **caractérisée en ce que** le milieu a la composition suivante :
| COMPOSITION | CONCENTRATION |
|---|---|
| Extrait autolytique de levures | 0,5 à 5 g/l |
| Peptone | 0,5 à 5 g/l |
| Glucose anhydre | 0,5 à 7 g/l |
| Micro-éléments de Heller | 0,5 à 5 ml/l |
| CaCl₂, 10 H₂O | 0,01 à 0,20 g/l |
| Eau distillée | qsp 1I |

12. Composition selon la revendication précédente, **caractérisée en ce que** les micro-éléments de Heller ont la composition suivante :
| | |
|---|---|
| ZnSO₄, 7 H₂O | 1g |
| MnSO₄, H₂O | 0,076 g |
| CuSO₄, 5H₂O | 0,003 g |
| KI | 0,010 g |
| H₃BO₃ | 1 g |
| AlCl₃, 6H₂O | 0,050 g |
| NiCl₂, 6H₂O | 0,030 g |
| Eau distillée | qsp 1I |

13. Composition selon l'une quelconque des revendications 11 ou 12, **caractérisée en ce que** la peptone est de la peptone papaïnique de soja.

14. Composition selon l'une quelconque des revendications précédentes, **caractérisée en ce que** la température de culture est comprise entre 18 et 40°C

15. Composition selon l'une quelconque des revendications précédentes, **caractérisée en ce que** le pH du milieu de culture est compris entre 5,5 et 8.

16. Composition cosmétique ou pharmaceutique, **caractérisée en ce qu'**elle comprend, dans un milieu cosmétiquement ou pharmaceutiquement acceptable, une quantité efficace de milieu de culture d'au moins une bactérie filamenteuse non photosynthétique tel que décrit dans les revendications 1 à 15 et au moins un produit à effet irritant.

17. Composition pharmaceutique selon l'une quelconque des revendications 1 à 16, destinée à diminuer et/ou retarder le vieillissement cutané.

18. Composition pharmaceutique selon l'une quelconque des revendications 1 à 16, destinée à stimuler le système immunitaire.

19. Composition pharmaceutique selon l'une quelconque des revendications 1 à 16, destinée à traiter les désordres associées à un excès de synthèse et/ou de libération de substance P.

20. Composition pharmaceutique selon l'une quelconque des revendications 1 à 16, destinée à traiter les désordres du système nerveux central, les troubles respiratoires, les syndromes allergiques, l'inflammation, la douleur, les désordres gastro-intestinaux, les désordres cutanés, les fibroses, les troubles de la maturation du collagène, les troubles cardio-vasculaires, les troubles vasospastiques, les désordres immunologiques et/ou les troubles du tractus urinaire.

21. Composition pharmaceutique selon l'une quelconque des revendications 1 à 16, destinée à traiter les peaux sensibles.

22. Composition pharmaceutique selon l'une quelconque des revendications 1 à 16, destinée à apaiser l'inflammation, à favoriser la cicatrisation, à diminuer l'acné et/ou la séborrhée et/ou à favoriser l'hydratation cutanée.

23. Procédé de traitement cosmétique destiné à diminuer et/ou à retarder le vieillissement cutané, **caractérisé par le fait que** l'on applique sur la peau, sur le cuir chevelu et/ou sur les muqueuses une composition cosmétique telle que décrite dans les revendications 1 à 16.

24. Utilisation d'une quantité efficace de milieu de culture d'au moins une bactérie filamenteuse non photosynthétique tel que décrit dans l'une des revendications 1 à 16 pour la préparation d'une composition cosmétique destinée à stimuler le système immunitaire, ladite composition étant appliquée sur la peau, sur le cuir chevelu et/ou sur les muqueuses.

25. Utilisation d'une quantité efficace de milieu de culture d'au moins une bactérie filamenteuse non photosynthétique tel que décrit dans les revendications 1 à 16 pour la préparation d'une composition cosmétique antagoniste de substance P.

26. Utilisation d'une quantité efficace de milieu de culture d'au moins une bactérie filamenteuse non photosynthétique tel que décrit dans l'une des revendications 1 à 16 pour la préparation d'une composition cosmétique destinée à traiter les désordres associées à un excès de synthèse et/ou de libération de substance P, ladite composition étant une composition cosmétique telle que décrite dans les revendications 1 à 16 pour application sur la peau, sur le cuir chevelu, et/ou sur les muqueuses.

27. Utilisation d'une quantité efficace de milieu de culture d'au moins une bactérie filamenteuse non photosynthétique tel que décrit dans l'une des revendications 1 à 16 pour la préparation d'une composition cosmétique destinée à traiter les peaux sensibles, ladite composition étant pour application sur la peau, sur le cuir chevelu et/ou sur les muqueuses.

28. Utilisation d'une quantité efficace de milieu de culture d'au moins une bactérie filamenteuse non photosynthétique tel que décrit dans l'une des revendications 1 à 16 pour la préparation d'une composition cosmétique telle que décrite dans les revendications 1 à 16 destinée à prévenir et/ou lutter contre les irritations cutanées et/ou les dartres et/ou les érythèmes et/ou les sensations dysesthésiques et/ou les sensations d'échauffement et/ou les prurits de la peau et/ou les muqueuses, par application sur la peau, sur le cuir chevelu et/ou sur les muqueuses.

29. Procédé de traitement cosmétique pour favoriser l'hydratation cutanée, **caractérisé en ce que** l'on applique sur la peau ou les muqueuses une composition cosmétique telle que décrite dans l'une des revendications 1 à 16.

30. Utilisation d'une quantité efficace de milieu de culture d'au moins une bactérie filamenteuse non photosynthétique tel que décrit dans l'une des revendications 1 à 16 pour la préparation d'une composition cosmétique destinée à apaiser l'inflammation, à favoriser la cicatrisation, à diminuer l'acné et/ou la séborrhée, ladite composition étant pour application sur la peau, sur les cheveux et/ou sur les muqueuses.

31. Milieu de culture d'au moins une bactérie filamenteuse non photosynthétique tel que décrit dans l'une des revendications 1 à 16, ledit milieu de culture ayant servi à l'amplification dudit micro-organisme et étant clarifié et stabilisé, pour son utilisation comme antagoniste de substance P.

## Patentansprüche

1. Kosmetische oder pharmazeutische Zusammensetzung, die in einem kosmetisch und/ oder pharmazeutisch akzeptablen Träger eine wirksame Menge eines Kulturmediums mindestens eines nichtphotosynthetischen filamentösen Bakteriums enthält, wobei das Medium zur Anreicherung des Mikroorganismus eingesetzt wurde und geklärt und stabilisiert ist.

2. Zusammensetzung nach dem vorhergehenden Anspruch, **dadurch gekennzeichnet, dass** das Bakterium zur Ordnung der Beggiatoales gehört.

3. Zusammensetzung nach einem der vorhergehenden Ansprüche, **dadurch gekennzeichnet, dass** das Bakterium zur Gattung *Beggiatoa, Vitreoscilla, Flexithrix* oder *Leucothrix* gehört.

4. Zusammensetzung nach einem der vorhergehenden Ansprüche, **dadurch gekennzeichnet, dass** das Bakterium unter den Stämmen von *Vitreoscilla filiformis* ausgewählt ist.

5. Zusammensetzung nach einem der vorhergehenden Ansprüche, **dadurch gekennzeichnet, dass** das Medium 0,0001 bis 30 % des Gesamtgewichts der Zusammensetzung ausmacht.

6. Zusammensetzung nach dem vorhergehenden Anspruch, **dadurch gekennzeichnet, dass** das Medium 0,01 bis 15 % des Gesamtgewichts der Zusammensetzung ausmacht.

7. Zusammensetzung nach einem der vorhergehenden Ansprüche, **dadurch gekennzeichnet, dass** das Medium durch Sterilfiltration, Autoklavieren, Ultrahocherhitzen, Hochdrucksterilisation, Bestrahlung mit γ-Strahlen oder Tiefkühlen stabilisiert wird.

8. Zusammensetzung nach dem vorhergehenden Anspruch, **dadurch gekennzeichnet, dass** das Medium durch Autoklavieren stabilisiert wird.

9. Zusammensetzung nach einem der vorhergehenden Ansprüche, **dadurch gekennzeichnet, dass** das Medium ein Acetat als Kohlenstoffquelle enthält.

10. Zusammensetzung nach einem der Ansprüche 1 bis 8, **dadurch gekennzeichnet, dass** das Medium eine Ose als Kohlenstoffquelle enthält.

11. Zusammensetzung nach einem der Ansprüche 1 bis 8 und 10, **dadurch gekennzeichnet, dass** das Medium die folgende Zusammensetzung aufweist:
| Zusammensetzung | Konzentration |
|---|---|
| Hefeextrakt, autolytisch | 0,5 bis 5 g/l |
| Pepton | 0,5 bis 5 g/l |
| wasserfreie Glucose | 0,5 bis 7 g/l |
| Heller-Mikroelemente | 0,5 bis 5 ml/l |
| CaCl₂, 10 H₂O | 0,01 bis 0,20 g/l |
| destilliertes Wasser | q.s.p. 1 l |

12. Zusammensetzung nach dem vorhergehenden Anspruch, **dadurch gekennzeichnet, dass** die Heller-Mikroelemente die folgende Zusammensetzung aufweisen:
| | |
|---|---|
| ZnSO₄, 7 H₂O | 1 g |
| MnSO₄, H₂O | 0,076 g |
| CuSO₄, 5 H₂O | 0,003 g |
| Kl | 0,010 g |
| H₃BO₃ | 1 g |
| AlCl₃, 6 H₂O | 0,050 g |
| NiCl₂, 6 H₂O | 0,030 g |
| destilliertes Wasser | q.s.p. 1 l |

13. Zusammensetzung nach einem der Ansprüche 11 oder 12, **dadurch gekennzeichnet, dass** das Pepton ein papainisch gewonnenes Pepton aus Soja ist.

14. Zusammensetzung nach einem der vorhergehenden Ansprüche, **dadurch gekennzeichnet, dass** die Kultivierungstemperatur im Bereich von 18 bis 40 °C liegt.

15. Zusammensetzung nach einem der vorhergehenden Ansprüche, **dadurch gekennzeichnet, dass** der pH-Wert des Kulturmediums im Bereich von 5,5 bis 8 liegt.

16. Kosmetische oder pharmazeutische Zusammensetzung, **dadurch gekennzeichnet, dass** sie in einem kosmetisch oder pharmazeutisch akzeptablen Medium eine wirksame Menge eines Kulturmediums mindestens eines nichtphotosynthetischen filamentösen Bakteriums nach einem der Ansprüche 1 bis 15 und mindestens ein Produkt mit irritierender Wirkung enthält.

17. Pharmazeutische Zusammensetzung nach einem der Ansprüche 1 bis 16, die dazu bestimmt ist, die Hautalterung zu vermindern und/oder zu verlangsamen.

18. Pharmazeutische Zusammensetzung nach einem der Ansprüche 1 bis 16, die dazu bestimmt ist, das Immunsystem zu stimulieren.

19. Pharmazeutische Zusammensetzung nach einem der Ansprüche 1 bis 16, die zur Behandlung von Erkrankungen bestimmt ist, die mit einer übermäßigen Synthese und/oder Freisetzung von Substanz P verbunden sind.

20. Pharmazeutische Zusammensetzung nach einem der Ansprüche 1 bis 16, die zur Behandlung von Erkrankungen des zentralen Nervensystems, Atemstörungen, allergischen Syndromen, Entzündung, Schmerz, gastrointestinalen Erkrankungen, Hautkrankheiten, Fibrosen, Störungen der Kollagenreifung, kardiovaskulären Störungen, vasospastischen Störungen, immunologischen Erkrankungen und/ oder Störungen des Harntrakts bestimmt ist.

21. Pharmazeutische Zusammensetzung nach einem der Ansprüche 1 bis 16, die für die Behandlung empfindlicher Haut vorgesehen ist.

22. Pharmazeutische Zusammensetzung nach einem der Ansprüche 1 bis 16, die dazu bestimmt ist, Entzündungen zu lindern, die Wundheilung zu begünstigen, Akne und/oder Seborrhoe zu vermindern und/oder die Hydratisierung der Haut zu fördern.

23. Verfahren zur kosmetischen Behandlung, das dazu bestimmt ist, die Hautalterung zu vermindern und/oder zu verlangsamen, **dadurch gekennzeichnet, dass** eine kosmetische Zusammensetzung nach einem der Ansprüche 1 bis 16 auf die Haut, die Kopfhaut und/oder die Schleimhäute angewendet wird.

24. Verwendung einer wirksamen Menge eines Kulturmediums mindestens eines nichtphotosynthetischen filamentösen Bakteriums nach einem der Ansprüche 1 bis 16 zur Herstellung einer kosmetischen Zusammensetzung, die zur Stimulation des Immunsystems bestimmt ist, wobei die Zusammensetzung auf die Haut, die Kopfhaut und/oder die Schleimhäute angewendet wird.

25. Verwendung einer wirksamen Menge eines Kulturmediums mindestens eines nichtphotosynthetischen filamentösen Bakteriums nach einem der Ansprüche 1 bis 16 zur Herstellung einer kosmetischen Zusammensetzung, die als Antagonist von Substanz P wirkt.

26. Verwendung einer wirksamen Menge eines Kulturmediums mindestens eines nichtphotosynthetischen filamentösen Bakteriums nach einem der Ansprüche 1 bis 16 zur Herstellung einer kosmetischen Zusammensetzung, die für die Behandlung von Erkrankungen vorgesehen ist, die mit einer übermäßigen Synthese und/oder Freisetzung von Substanz P verbunden sind, wobei es sich bei der Zusammensetzung um eine kosmetische Zusammensetzung nach einem der Ansprüche 1 bis 16 zur Anwendung auf die Haut, die Kopfhaut und/oder die Schleimhäute handelt.

27. Verwendung einer wirksamen Menge eines Kulturmediums mindestens eines nichtphotosynthetischen filamentösen Bakteriums nach einem der Ansprüche 1 bis 16 zur Herstellung einer kosmetischen Zusammensetzung, die zur Behandlung von empfindlicher Haut bestimmt ist, wobei die Zusammensetzung auf die Haut, die Kopfhaut und/oder die Schleimhäute anzuwenden ist.

28. Verwendung einer wirksamen Menge eines Kulturmediums mindestens eines nichtphotosynthetischen filamentösen Bakteriums nach einem der Ansprüche 1 bis 16 zur Herstellung einer kosmetischen Zusammensetzung nach einem der Ansprüche 1 bis 16, mit der durch Anwendung auf die Haut, die Kopfhaut und/oder die Schleimhäute Hautirritationen und/oder Flechten und/oder Erytheme(n) und/oder dysästhesische(n) Empfindungen und/oder Hitzegefühl und/oder Juckreiz der Haut und/oder der Schleimhäute vorgebeugt und/oder bekämpft werden sollen.

29. Verfahren zur kosmetischen Behandlung, um die Hydratisierung der Haut zu fördern, **dadurch gekennzeichnet, dass** eine kosmetische Zusammensetzung nach einem der Ansprüche 1 bis 16 auf die Haut oder die Schleimhäute angewendet wird.

30. Verwendung einer wirksamen Menge eines Kulturmediums mindestens eines nichtphotosynthetischen filamentösen Bakteriums nach einem der Ansprüche 1 bis 16 zur Herstellung einer kosmetischen Zusammensetzung, die zur Linderung von Entzündungen, zur Förderung der Wundheilung, zur Minderung von Akne und/ oder von Seborrhoe bestimmt ist, wobei die Zusammensetzung auf die Haut, die Haare und/ oder die Schleimhäute anzuwenden ist.

31. Kulturmedium mindestens eines nichtphotosynthetischen filamentösen Bakteriums nach einem der Ansprüche 1 bis 16 zur Verwendung als Antagonist von Substanz P, wobei das Kulturmedium zur Anreicherung des Mikroorganismus eingesetzt wurde und geklärt und stabilisiert ist.

## Claims

1. Cosmetic or pharmaceutical composition comprising, in a cosmetically and/or pharmaceutically acceptable support, an effective amount of a culture medium for at least one non-photosynthetic filamentous bacterium, the said medium having been used for amplification of the said microorganism and being clarified and stabilized.

2. Composition according to the preceding claim, **characterized in that** the said bacterium belongs to the order of Beggiatoales.

3. Composition according to either of the preceding claims, **characterized in that** the said bacterium belongs to the genus *Beggiatoa, Vitreoscilla, Flexithrix* or *Leucothrix.*

4. Composition according to any one of the preceding claims, **characterized in that** the said bacterium is chosen from strains of *Vitreoscilla filiformis*.

5. Composition according to any one of the preceding claims, **characterized in that** the medium represents from 0.0001% to 30% of the total weight of the composition.

6. Composition according to the preceding claim, **characterized in that** the medium represents from 0.01% to 15% of the total weight of the composition.

7. Composition according to any one of the preceding claims, **characterized in that** the medium is stabilized by sterilizing filtration, autoclaving, ultra-high temperature, high-pressure sterilization, γ-radiation or freezing.

8. Composition according to the preceding claim, **characterized in that** the medium is stabilized by autoclaving.

9. Composition according to any one of the preceding claims, **characterized in that** the medium comprises an acetate as a carbon source.

10. Composition according to any one of Claims 1 to 8, **characterized in that** the medium comprises a saccharide as a carbon source.

11. Composition according to any one of Claims 1 to 8 and 10, **characterized in that** the medium has the following composition:
| COMPOSITION | CONCENTRATION |
|---|---|
| Autolytic yeast extract | 0.5 to 5 g/l |
| Peptone | 0.5 to 5 g/l |
| Anhydrous glucose | 0.5 to 7 g/l |
| Heller microelements | 0.5 to 5 ml/l |
| CaCl₂A10H₂O | 0.01 to 0.20 g/l |
| Distilled water | qs 1 l |

12. Composition according to the preceding claim, **characterized in that** the Heller microelements have the following composition:
| | |
|---|---|
| ZnSO₄·7H₂O | 1 g |
| MnSO₄·H₂O | 0.076 g |
| CuSO₄·5H₂O | 0.003 g |
| KI | 0.010 g |
| H₃BO₃ | 1 g |
| AlCl₃ 6H₂O | 0.050 g |
| NiCl₂·6H₂O | 0.030 g |
| Distilled water | qs 1 l |

13. Composition according to either of Claims 11 and 12, **characterized in that** the peptone is soybean papain peptone.

14. Composition according to any one of the preceding claims, **characterized in that** the culturing temperature is between 18 and 40°C.

15. Composition according to any one of the preceding claims, **characterized in that** the pH of the culture medium is between 5.5 and 8.

16. Cosmetic or pharmaceutical composition, **characterized in that** it comprises, in a cosmetically or pharmaceutically acceptable medium, an effective amount of a culture medium for at least one non-photosynthetic filamentous bacterium, as described in Claims 1 to 15, and at least one product with an irritant effect.

17. Pharmaceutical composition according to any one of Claims 1 to 16, intended to reduce and/or delay ageing of the skin.

18. Pharmaceutical composition according to any one of Claims 1 to 16, intended to stimulate the immune system.

19. Pharmaceutical composition according to any one of Claims 1 to 16, intended to treat disorders associated with excessive synthesis and/or release of substance P.

20. Pharmaceutical composition according to any one of Claims 1 to 16, intended to treat disorders of the central nervous system, respiratory disorders, allergic syndromes, inflammation, pain, gastrointestinal disorders, skin disorders, fibrosis, disorders of collagen maturation, cardiovascular disorders, vasospastic disorders, immunological disorders and/or disorders of the urinary tract.

21. Pharmaceutical composition according to any one of Claims 1 to 16, intended to treat sensitive skin.

22. Pharmaceutical composition according to any one of Claims 1 to 16, intended to soothe inflammation, to promote cicatrization, to reduce acne and/or seborrhoea and/or to promote moisturization of the skin.

23. Cosmetic treatment process intended to reduce and/or delay ageing of the skin, **characterized in that** a cosmetic composition as described in Claims 1 to 16 is applied to the skin, to the scalp and/or to the mucous membranes.

24. Use of an effective amount of a culture medium for at least one non-photosynthetic filamentous bacterium, as described in one of Claims 1 to 16, for the preparation of a cosmetic composition intended to stimulate the immune system, the said composition being applied to the skin, to the scalp and/or to the mucous membranes.

25. Use of an effective amount of a culture medium for at least one non-photosynthetic filamentous bacterium, as described in Claims 1 to 16, for the preparation of a cosmetic composition which is a substance P antagonist.

26. Use of an effective amount of a culture medium for at least one non-photosynthetic filamentous bacterium, as described in one of Claims 1 to 16 for the preparation of a cosmetic composition intended to treat disorders associated with excessive synthesis and/or release of substance P, the said composition being a cosmetic composition as described in Claims 1 to 16 for application to the skin, to the scalp and/or to the mucous membranes.

27. Use of an effective amount of a culture medium for at least one non-photosynthetic filamentous bacterium, as described in one of Claims 1 to 16 for the preparation of a cosmetic composition intended to treat sensitive skin, the said composition being for application to the skin, to the scalp and/or to the mucous membranes.

28. Use of an effective amount of a culture medium for at least one non-photosynthetic filamentous bacterium, as described in one of Claims 1 to 16 for the preparation of a cosmetic composition as described in Claims 1 to 16 intended to prevent and/or combat skin irritation and/or dry patches and/or erythema and/or dysaesthesic sensations and/or heating sensations and/or pruritus of the skin and/or the mucous membranes, by application to the skin, to the scalp and/or to the mucous membranes.

29. Cosmetic treatment process to promote moisturization of the skin, **characterized in that** a cosmetic composition as described in one of Claims 1 to 16 is applied to the skin or mucous membranes.

30. Use of an effective amount of a culture medium for at least one non-photosynthetic filamentous bacterium, as described in one of Claims 1 to 16 for the preparation of a cosmetic composition intended to soothe inflammation, to promote cicatrization, to reduce acne and/or seborrhoea,the said composition being for application to the skin, to the hair and/or to the mucous membranes.

31. Culture medium for at least one non-photosynthetic filamentous bacterium as described in one of Claims 1 to 16, the said culture medium having been used for amplification of the said microorganism and being clarified and stabilized, for its use as a substance P antagonist.
